Europäisches Patentamt

European Patent Office

Office européen des brevets

(11) Publication number: **0 245 993 B1**

# EUROPEAN PATENT SPECIFICATION

(45) Date of publication of patent specification: 26.05.93

(51) Int. Cl.5: **C12P 21/08**, A61K 39/40, G01N 33/577

(21) Application number: 87303762.6

(22) Date of filing: 28.04.87

(54) Monoclonal antibodies against C5A and DES−ARG74−C5A, their production and use.

(30) Priority: 28.04.86 US 856780
30.12.86 US 947839

(43) Date of publication of application:
19.11.87 Bulletin 87/47

(45) Publication of the grant of the patent:
26.05.93 Bulletin 93/21

(84) Designated Contracting States:
AT BE CH DE ES FR GB GR IT LI LU NL SE

(56) References cited:
WO−A−84/04458

COMPLEMENT, vol. 3, no. 1, 1986, pages
25−39, Karger AG, Basel, CH; M. SCHULZE et
al.: "A sensitive ELISA for the quantitation of
human C5a in blood plasma using a mon-
oclonal antibody"

THE JOURNAL OF BIOLOGICAL CHEMISTRY,
vol. 260, no. 18, 25th August 1985, pages
10339−10345; R.J. JOHNSON et al.:
"Structure and function of human C5a an−
aphylatoxin"

(73) Proprietor: **CETUS ONCOLOGY CORPORA−TION**
**1400 Fifty−Third Street**
**Emeryville California 94608(US)**

(72) Inventor: **Larrick, James W.**
**Star Rt. Box 48**
**Woodside California 94062(US)**
Inventor: **Fendly, Brian M.**
**49 Showers Drive**
**Mountain View California 94040(US)**
Inventor: **Deinhart, Tracey E.**
**679 Blackford Court**
**San Jose California 94117(US)**

(74) Representative: **Bizley, Richard Edward et al**
**HEPWORTH LAWRENCE BRYER & BIZLEY**
**2nd Floor Gate House South West Gate**
**Harlow, Essex CM20 1JN (GB)**

JOURNAL OF IMMUNOLOGICAL METHODS, vol. 62, 1983, pages 305–314, Elsevier Science Publishers B.V.; S.L. KUNKEL et al.: "A specific enzyme–linked immunosorbent assay (ELISA) for the determination of human C5a antigen"

CLINICAL RESEARCH, vol. 35, no. 1, 1987, page 140A; J.W. LARRICK et al.: "Neutralizing anti–human C5a monoclonal antibodies"

## Description

This invention relates to monoclonal antibodies which are found to bind specifically to the human C5a glycoprotein or its des − arg derivative and to block the adverse biological effects of human C5a and its des − arg derivative. This invention also relates to methods of treatment using such antibodies and immunological and immunodiagnostic methods.

Human C5a is a glycoprotein with a molecular weight of 11,300 daltons, of which 8,285 daltons is polypeptide and the remainder is carbohydrate. Hugli and Muller − Eberhard, Adv. Immunol., 26:1 (1978). C5a is generated as a cleavage product of complement protein C5 in both the classical and alternative pathways of complement activation.

Human C5a possesses anaphylatoxic, chemotactic, contractile, and permeability − enhancing activities. Hugli and Muller − Eberhard, supra. The anaphylatoxic activity of human C5a has been studied extensively, and C5a has been shown to release maximal amounts of histamine from mast cells and basophils. C5a is rapidly converted in vivo to des − arg75 − C5a by carboxypeptidase B in serum. Bokisch et al., J. Exp. Med., 129:1109 (1969). This C5a derivative possesses greatly reduced anaphylatoxic, contractile, and permeability − enhancing activity, while retaining potent chemotactic activity. The carbohydrate moiety of human C5a, which is attached to the side chain of asparagine − 64, is also thought to modulate its anaphylatoxic, contractile, and permeability − enhancing activities. Gerard and Hugli, PNAS (USA), 78:1833 (1981).

A specific C5a receptor has been identified on human neutrophils. The chemotactic response of human neutrophils to purified human C5a has been observed at concentrations of 0.4 − 17 nM. Chenoweth and Hugli, Mol. Immunol., 17:151 (1980). This response diminishes at higher concentrations. In neutrophils isolated from burn patients, a temporal, specific loss of chemotactic response is associated with a rise in immunoreactive C5a after postburn day 4.

Neutrophil adherence to vascular endothelial cells is the initial event in the migration of neutrophils through blood vessel walls to localize at tissue sites of inflammation. C5a − stimulated and des − arg74 − C5a − stimulated neutrophil adherence to cells or to protein − coated plastic occurs rapidly, and neutrophils exposed to C5a self − aggregate.

C5a has been shown to be the causal agent of complement − induced granulocyte aggregation. Craddock et al, J. Clin. Invest., 60:260 (1977). Such aggregation in vivo has been postulated as a mechanism of tissue damage in such clinical conditions as pulmonary dysfunction and leukostasis in hemodialysis patients, sudden blindness with retinal infarction after trauma or pancreatitis, myocardial infarction, postpump syndrome after cardiopulmonary bypass surgery, systemic lupus erythematosus (SLE), adult respiratory distress syndrome (ARDS), burn injury and lung injury.

Strategies to block the interaction between C5a and neutrophils include treatment with high doses of corticosteroids. O'Flaherty et al., Proc. Soc. Exp. Biol. Med., 154:206 (1977).

Recently, Bumpers and Baum, J. Lab. Clin. Med., 102:421 (1983) disclosed a novel C5 inhibitor effective in blocking formation of a chemotactic factor for cultured Walker carcinosarcoma cells. However, such compound is unlikely to be effective in neutralizing C5a, because addition of the drug after complement activation had no effect on tumor cell chemotaxis.

An article by Stevens et al., "Effect of anti − C5a Antibodies on the Adult Respiratory Distress Syndrome in Septic Primates", a preprint in 1985 of J. Clin. Invest., 77(6):1812 − 1816 (June 1986), entitled "Effects of Anti − C5a Antibodies on the Adult Respiratory Distress Syndrome in Septic Primates", used leucocyte aggregometry to show that adding polyclonal rabbit anti − human des − arg74 − C5a to endotoxin − activated plasma prevented leukocyte aggregation in vitro. In addition, Upjohn Laboratories markets radioimmunoas − say kits including polyclonal antibodies for human C5a, and Kunkel et al., J. Imm. Methods, 62:305 (1983) discloses a specific ELISA for C5a using polyclonal antibodies.

Polyclonal antibodies to C5 (which is larger than C5a and present at much higher concentrations in the serum) are available commercially from several sources. Furthermore, C5 monoclonal antibodies are commercially available. However, it is the C5a fragment, rather than the C5, which is to be inhibited because only C5a causes chemotaxis and neutrophil aggregation.

Finally, Chenoweth et al., J. Biol. Chem., 260:10339 − 10345 (August, 1985) states that a murine anti − human C5a monoclonal antibody was used which cross − reacted exclusively with a C5a tyrosyl residue in C5a.

Because C5a has no known enzymatic activity, specific binding thereto and direct neutralization thereof would appear to represent the best approach to combat the toxicity of the C5a mediator.

Accordingly, the present invention provides a monoclonal antibody which specifically binds to (is reactive with) human complement component C5a or des − arg74 − C5a. The antibody herein blocks the binding of said C5a or des−arg74−C5a to human granulocytes and blocks the effect of C5a or des− arg74−C5a in vivo. Preferably the antibody has an affinity of at least $10^8$ liters/mole for human C5a or human des−arg74−C5a.

In another aspect, the invention provides stable, permanent hybrid cell lines which produce the above described antibody and progeny thereof.

In a further aspect, the invention provides a composition for treating a condition associated with (or caused by) injurious intravascular complement activation comprising a therapeutically effective amount of the above described antibody in association with a pharmaceutically or veterinarily acceptable vehicle. One or more of the antibodies or the composition may be administered in an effective amount to treat a mammalian patient.

In yet another aspect, the invention relates to a composition for treating Gram−negative sepsis comprising a therapeutically effective amount of a mixture of at least one of the above described antibodies and at least one antibody which is reactive with Gram−negative bacterial endotoxin, said mixture of antibodies being in association with a pharmaceutically or veterinarily acceptable vehicle. This composition may be administered in an effective amount to a mammalian patient for treatment of Gram−negative sepsis.

The invention herein also relates to an improved immunologic or immunodiagnostic method and composition for detecting human C5a or human des−arg74−C5a in fluids using at least in part at least one of the above−described antibodies to human C5a or human des−arg74−C5a.

In another aspect the invention a method of producing an antibody of the invention which comprises culturing a hybridoma as referred to above. Also, in general the invention provides a method of producing a medicament which comprises formulating for pharmaceutical or veterinary use, optionally together with an acceptable vehicle, an antibody as defined above.

The monoclonal antibodies of this invention fulfill many critical requirements not satisfied by existing polyclonal or monoclonal antibody technology. Similar to polyclonal antibodies to C5a, such monoclonal antibodies are ideally cross−reactive among C5a from diverse species so as to have potential utility to treat non−human mammals. Such monoclonal antibodies, however, would be more useful than polyclonal antibodies as immunoprophylactic, therapeutic and diagnostic reagents because of their unique specificity and affinity. The specificity also renders the monoclonal antibodies more useful for immunological and biochemical studies of human C5a and its des−arg derivative, for affinity purification of C5a or des− arg74−C5a, and for the neutralization and/or removal of C5a or des−arg74−C5a from any reagents where it might be present. Furthermore, unlike conventional polyclonal antibodies, monoclonal antibodies reactive with C5a or des−arg74−C5a can be produced in a potentially limitless and homogeneous supply so as to achieve uniform, consistent results.

Finally, the monoclonal antibodies herein block the binding of C5a or des−arg74−C5a to human granulocytes, and bind C5a or des−arg74−C5a in the presence of a molar excess of complement component C5. In contrast, the polyclonal antibodies to C5a are not able to bind the C5a component in serum, which typically contains a greater than 100−fold molar excess of C5.

As used herein, the term "fluids" refers to biological fluids such as serum, urine, saliva, tears, sputum, perspiration, etc., or process fluids such as culture media, fermentation broth, etc., which are suspected of containing or known to contain human C5a or des−arg74−C5a.

As used herein, the term "injurious intravascular complement activation" refers to any process in the complement system whereby the mediator human C5a or des−arg74−C5a is released so as to be injurious to the health of the individual in whom it is released. Conditions associated with such activation include pathogenic conditions such as inflammatory diseases, Gram−negative infections, autoimmune diseases, etc., as well as trauma or injuries.

As used herein, the term "self−reproducing carrier cell containing genes coding for and specifying production of the antibody" refers to any self−replicating cell line which can produce the antibodies, including a microorganism, a virus−transformed cell line or a hybridoma. A hybridoma is meant to include triomas from fusion of a mouse x human hybrid with Epstein−Barr virus−transformed peripheral blood lymphocytes or splenocytes, as well as hybrids of a myeloma cell line with splenocytes.

As used herein, the term "cell line" refers to individual cells, harvested cells, and cultures containing cells so long as they are derived from cells of the cell line referred to.

As used herein with respect to hybrid cell lines, the term "progeny" is intended to include all derivatives, issue, and offspring of the cell lines regardless of generation of karyotypic identity.

As used herein with respect to a given antibody, the term "functional equivalent" means an antibody that recognizes the same determinant as and crossblocks the antibody referred to. It is intended to include antibodies of the same or different immunoglobulin class and antigen binding fragments (e.g., Fab, F(ab')₂,

Fv) of the antibody.

As used herein with respect to administering antibody to patients, the term "treat" and conjugates thereof refers to therapy and/or prophylaxis.

As used herein, the term "monoclonal antibody" refers to an antibody selected from antibodies whose population is substantially homogenous, i.e., the individuals of the antibody population are identical except for naturally occurring mutations.

As used herein with respect to characterizing the claimed hybrid cell lines, the terms "permanent" and "stable" mean that the lines remain viable over a prolonged period of time, typically at least about six months, and maintain the ability to produce the specified monoclonal antibody through at least about 50 generations.

Monoclonal antibodies which meet the functional and preferred criteria of the invention (specific binding to C5a or the des − arg derivative, blocking, and affinity) may be made using cells of diverse mammalian origin, including mouse, rat, rabbit, porcine, primate and human embodiments. The antibodies may be of any isotype, including IgG and IgM.

The human embodiments of the antibodies herein may be the products of triomas synthesized by somatic cell hybridization using a mouse x human parent hybrid cell line and Epstein − Barr virus (EBV) − transformed human peripheral blood lymphocytes (PBL) or splenocytes from non − immunized volunteers or volunteers immunized with available human C5a or its des − arg derivative. Fresh PBLs or spenocytes (not transformed) may be used, if desired. Preferably the human C5a is purified before immunization.

The mouse embodiments may be prepared by fusing in the presence of a fusogen such as polyethylene glycol a mouse myeloma line with splenocytes from mice immunized with C5a or its des − arg derivative to produce a hybridoma, using the somatic cell hybridization method first described by Kohler and Milstein, Eur. J. Immunol., 6:511 − 519 (1976).

The antibodies are generated by standard methods (e.g., Oi and Herzenberg, in Selected Methods in Cellular Immunology, B. Mishell et al., et., W. J. Freeman Co., San Francisco, p. 351 − 371 (1980), and hybridomas selected in an appropriate selection medium by standard techniques, e.g., Foung, S. K. H. et al. (1983), Proc. Natl. Acad. Sci., 79:7484 − 7488.

The positive hybrids selected are characterized for their ability to react with (bind specifically to) C5a or des − arg74 − C5a. This can be determined by ascertaining the ability of the antibodies to immunoprecipitate $^{125}$I − labeled C5a or des − arg74 − C5a, to bind C5a or des − arg74 − C5a in solid phase EIA, and to recognize peptide fragments of C5a or des − arg74 − C5a.

The positive hybrids are tested for their affinity for C5a or des − arg74 − C5a by standard methods such as solid phase radioimmunoassay. An affinity constant of at least $10^8$ liters/mole is preferable to obtain the best effect.

In addition, the antibodies are characterized for their epitopes and isotypes. They are also tested for whether they cross − react with C5a produced in animal species other than humans, such as dog, pig, guinea pig and monkey C5a.

The antibodies are also tested for their ability to neutralize human C5a or des − arg74 − C5a, i.e., their ability to block the binding of human C5a or des − arg74 − C5a to human granulocytes, regardless of the particular mechanism involved. It is intended to include, without limitations, mechanisms in which the antibody affects the biological activity of the human C5a or des − arg74 − C5a by binding thereto, causes the C5a or des − arg74 − C5a to be degraded, or affects the activity of the C5a or des − arg74 − C5a by altering the kinetics and/or site of its clearance. Neutralizing activity may be shown, for example, by the ability of the antibody to block $^{125}$I − C5a binding to polymorphonuclear leukocytes, to generate superoxide anion, to induce neutrophil chemotaxis, and to stimulate neutrophil reduction of nitrobluetetrazolium dye (NBT).

Finally, the antibodies herein are tested for their ability to bind human C5a or des − arg75 − C5a in the presence of a molar excess of complement component C5 by the following test. Human and other animal sera are activated by adding thereto yeast cell wall fragments which promote cleavage of C5 to C5a and/or des − arg74 − C5a. The inhibition of $^{125}$I − C5a immunoprecipitation in the presence of activated and unac − tivated serum is then measured for the antibody supernatant at various concentrations.

The hybridomas which produce the antibodies of this invention may be grown in suitable culture media such as Iscove's media or RPMI − 1640 medium or in vivo in laboratory animals. If desired, the antibody may be separated from the culture medium or body fluid, as the case may be, by conventional techniques such as ammonium sulfate precipitation, hydroxylapatite chromatography, ion exchange chromatography, affinity chromatography, electrophroesis, microfiltration and ultracentrifugation.

The antibodies of this invention may be used passively to immunize human individuals (or other mammalian species) who suffer from a condition associated with injurious intravascular complement activation or those who are at risk with respect to this condition, including patients receiving immunosup −

pressive therapy and those suffering from severe thermal burns or other serious injuries. Examples of conditions associated with intravascular complement activation include, but are not limited to, Gram – negative sepsis, ARDS, thermal injury, pulmonary inflammation or injury, severe trauma, pancreatitis, myocardial infarction, massive blood transfusion, blood clots, cardiovascular disease, exposure to medical devices (including but not limited to hemodialyzer membranes and extracorporeal blood circulation equip – ment), and/or acute phases of chronic autoimmune disease (including but not limited to systemic lupus erythrematosus and rheumatoid arthritis).

In addition, a combination of antibiotic and antiinflammatory agent such as corticosteroids (e.g., methylprednisolone) and one or more of the above – described antibodies may be employed. Also, one or more of the above – described antibodies may be used in combination with one or more monoclonal antibodies against Gram – negative bacterial endotoxin to treat Gram – negative sepsis.

These latter antibodies may be obtained as described in PCT WO84/04458 published November 22, 1984 and PCT WO84/01643 published April 25, 1985. The antibodies may act synergistically in that the endotoxin – specific antibody may neutralize or hasten C5a clearance while the C5a – specific antibody may neutralize the toxin.

The antibodies herein may also be used immunologically or immunodiagnostically to detect the presence or absence of human C5a or human des – arg74 – C5a in fluids such as blood, urine, etc. The fluid may be incubated in the presence of at least one of the antibodies described herein. The presence or absence and/or degree of reaction can be determined by any of a variety of methods used to determine or quantitate antibody/antigen interaction (e.g., hemagglutination, latex agglutination, complement fixation, radioimmunoassay, enzyme immunoassay, fluorescent immunoassay, sandwich assay, fluorescent micro – scopy, etc.). For example, a sandwich immunoassay may be used wherein the test sample is incubated with a first monoclonal antibody directed against one epitope of the antigen which is immobilized on a solid support such as a plastic tube or polystyrene beads and the test sample is incubated with a second monoclonal antibody directed against a different epitope of the antigen which is labeled with a detectable moiety which can be detected, for example, immunologically, enzymatically, by use of a peptide, or by spectrophotometric, chemical or radiological means. The incubation with the immobilized antibody may take place before, during or after incubation with the labeled antibody. The antibody may be detected indirectly by reaction with another ligand (typically a monoclonal antibody which is specific thereto) which is labeled with a detectable moiety.

The antibodies may be administered to a mammalian patient by any suitable technique, including subcutaneous and parenteral administration, preferably parenteral. Examples of parenteral administration include intravenous, intraarterial, intramuscular and intraperitoneal, preferably intravenous. The dose and dosage regimen will depend mainly upon whether the antibody/antibodies is/are being administered for therapeutic or prophylactic purposes, the patient, and the patient's history. The total pharmaceutically effective amount of an antibody administered per dose will typically be in the range of about 0.2 to 20 mg/kg of patient body weight.

For parenteral administration the antibody/antibodies will generally be formulated in a unit dosage injectable form (solution, suspension, emulsion) in association with a pharmaceutically acceptable parenteral vehicle. Such vehicles are inherently nontoxic and nontherapeutic. Examples of such vehicles include water, saline, Ringer's solution, dextrose solution, and 5% human serum albumin. Nonaqueous vehicles such as fixed oils and ethyl oleate may also be used. Liposomes may be used as carriers. The vehicle may contain minor amounts of additives such as substances which enhance isotonicity and chemical stability, e.g., buffers and preservatives. The antibody will typically be formulated in such vehicles at a concentration of about 0.1 mg/ml to 100 mg/ml.

The various aspects of the invention are further described by the following examples, which are not intended to limit the invention in any manner. In these examples all percentages for solids are by weight and all percentages for liquids and gases are by volume unless otherwise noted, and all temperatures are given in degrees Celsius.

## EXAMPLE 1

### Monoclonal Antibody Production

Balb/c mice were initially injected intraperitoneally with 3 – 10 $\mu$g of purified human C5a (from Dr. Steven Kunkel of University of Michigan at Ann Arbor, MI) in complete Freund's adjuvant (commercially obtained). After two weeks the mice were injected intraperitoneally with 3 – 10 $\mu$g of the same purified C5a in Incomplete Freund's adjuvant. Following weekly intraperitoneal boosts of the C5a in phosphate buffered

EP 0 245 993 B1

saline (PBS), the last injection consisted of an intravenous injection with 4 μg of purified C5a in PBS. Three days after the final immunization, the mice were sacrificed, and their spleens were removed. The spleen cells were suspended in Dulbecco's Modified Eagle's Medium (DMEM) and fused to mouse myeloma cells SP2/OAg14 (available commercially and deposited with the American Type Culture Collection (ATCC), Rockville, MD, as ATCC No. CRL1581) in a ratio of spleen to myeloma of 5:1 by cell number, using polyethylene glycol of molecular weight 1000 as fusogen.

The fusion product was plated in a microtiter plate well containing a solution of hypoxanthine and azaserine as described by Larrick et al., in Kennett et al. (eds), Monoclonal Antibodies and Hybridomas:Progress and Applications (New York:Plenum, 1984). Positive clones in the plate well were then grown in mass culture or murine ascites.

Multi – Well Immunoprecipitation

Hybridoma supernatants were then screened for positive clones by immunoprecipitation. Wells of a 96 – well polyvinyl chloride microtiter plate were blocked with 1% bovine serum albumin in phosphate buffered saline (PBS), pH 7.2 for 60 minutes at 37˚C. The plates were then emptied of blocking solution, and 50 μl of the supernatant or purified antibody was added to each well. Then 100,000 cpm of $^{125}$I – labeled human C5a (50 μl) was added to each well, and the mixture was incubated for 60 minutes at room temperature. Following this incubation, 50 μl of a 1:5 dilution of rabbit anti – mouse immunoglobulin immobilized to a plastic carrier bead (immunobeads) commercially obtained was added to each well and incubated for 60 minutes at room temperature with agitation. The immunobeads were then washed three times with a solution of PBS and 0.1% Tween 20 (hereinafter "PBST") and the plates were cut. The individual wells were counted on a gamma counter.

After counting, the immunobeads were resuspended in 30 μl of sample buffer (0.06 M Tris – HCl, pH 7.0, 5% 2 – mercaptoethanol, 2% SDS, 1.5% glycerol). This suspension was boiled for three minutes and then the supernatant was analyzed by SDS – PAGE, according to the stacking gel procedure of Laemmli, Nature (London) 227:680 – 685 (1970). The gels were dried and exposed to Cronex X – ray film at – 70˚C with intensifying screens, as described by Bonner and Laskey, Eur. J. Biochem., 46:83 – 88 (1974).

Affinity Determination

The fluid containing the antibodies was titrated in doubling dilutions and 200 μl titrated antibody was then placed in 12 x 75 mm polypropylene tubes. A total of 100,000 cpm (100 μl) of $^{125}$I – labeledC5a was then added to the samples. Samples were then mixed and incubated for two hours at room temperature. A total of 100 μl of a 1:5 dilution of rabbit anti – mouse immunoglobulin immunobeads was added to each tube and incubated for 60 minutes at room temperature and washed twice with 4 ml PBS. Then the mixtures were centrifuged at 2800 rpm for 10 minutes at 4˚C and the tubes were counted on a gamma counter. Results were analyzed and 75% maximal binding was determined.

The fluid was diluted to its appropriate concentration (75% maximum binding) with 5% BSA in PBS. Unlabeled human C5a was titrated in doubling dilutions and 200 μl of the titrated unlabeled C5a plus 200 μl of the monoclonal supernatant was mixed in 12 x 75 mm polypropylene tubes for two hours at room temperature. A total of 100,000 cpm of $^{125}$I – labeled C5a (100 μl) was then added to each sample, mixed, and incubated for one hour at room temperature. A total of 100 μl of a 1:5 dilution of rabbit anti – mouse immunoglobulin immunobeads was added to each sample and incubated for 60 minutes at room tempera – ture. Samples were then washed two times with 4 ml PBS, centrifuged at 2800 rpm for 10 minutes at 4˚C and counted on a gamma counter. A total of 50% inhibition of binding was determined and the affinity constant was then calculated according to the method of Mueller, J. Immunol. Methods, 34:345 – 352 (1980). Affinity as determined by the above asay is reported in Table I as Affinity (ℓ/mole)A.

Affinity was determined a second time in essentially the same manner as above except that con – centrations of cold C5a ranged from 8 ng to 10 μg/ml in 200 μl PBS, 5% BSA. The affinity of the monoclonal antibody is determined according to this procedure and is reported in Table I as Affinity (ℓ/mole)B.

Antibody Purification

Tetramethylpentadecane primed Balb/c mice were injected intraperitoneally with 5 x 10$^6$ of the selected hybridoma cells. Ascites fluid was collected 14 – 28 days later, centrifuged, and stored at – 76˚C until needed. Ascites fluid was filtered and applied to a protein A monoclonal antibody column purification

7

system (MAPs). Active fractions were collected and dialyzed against PBS. Fractions were then filtered through a 0.2 $\mu$m filter and stored in the same buffer at 4°C.

Epitope Analysis

The epitopes of the antibodies may be determined by the following procedure. Wells of a 96−well polyvinyl chloride microtiter plate are coated with 50 $\mu$l/well of 1 $\mu$g/ml purified human C5a and incubated for 60 minutes at 37°C. The plates are then emptied and wells are blocked with 1% BSA in PBS, pH 7.2 for 60 minutes at 37°C. Then the plates are washed three times with PBST. A total of 50 $\mu$l of the monoclonal antibodies which are purified as described above at various concentrations are added to wells and incubated for 60 minutes at room temperature. Fifty $\mu$l/well of 1:1000 dilution of peroxidase−labeled monoclonal antibody against human C5a is added directly to the wells and incubated for 60 minutes at room temperature. Such peroxidase−labeled monoclonal antibodies are conjugated according to the method of Nakane and Kawaoi, J. Histochem. Cytochem., 22:1084 (1974). Plates are washed three times in PBST. A total of 0.1 ml/well o−phenylenediamine is added as developer and the reaction is stopped by adding 0.1 ml/well 1 N HCl. Plates are read at 492 nm on a multiscan spectrophotometer.

Isotype Determination

Isotype determination was performed as follows. Briefly, a 96−well polyvinyl chloride plate was coated with purified human C5a and then blocked with 1% BSA in PBS, pH 7.2. Then 50 $\mu$l of the culture supernatant described above was added to the wells and incubated for 60 minutes at room temperature. The plates were washed and 50 $\mu$l of each subclass−specific rabbit anti−mouse Ig antibody was added to separate wells. After an incubation for 60 minutes at room temperature and a 3X wash, 50 $\mu$l of peroxidase−labeled goat anti−rabbit IgG was added to each well, and incubated for 60 minutes at room temperature. Plates were washed three times and 0.1 ml of 2,2−azino−bi(3−ethylbenzthiazoline sulfonic acid) solution was added to each well and the reaction stopped by adding 0.1 ml of 0.1 N HCl. Results were read with a multiscan spectrophotometer.

Neutrophil Preparation and Polarization

Human polymorphonuclear leukocytes (PMNs) were isolated by diluting heparinized blood with 6% dextran (5:1, blood:dextran). The red blood cells were allowed to settle 45 minutes at room temperature.

The white blood cell−enriched plasma was removed and diluted 1:2 with Hank's Balanced Salt Solution (HBSS). This was layered over Ficoll−Hypaque and centrifuged for 20 minutes at 1600 rpm at room temperature.

All but the pellet was discarded and the cells were resuspended in HBSS at 1.25 x $10^6$ cells/ml. These were stored on ice until used.

A total of 0.1 ml of stimulant (stocks were 10 x the desired concentration) to be tested was added to 12 x 75 mm polypropylene tubes. These were run in duplicates. The negative control had 0.1 ml HBSS only; the positive control had 0.1 ml n−formaldehyde−L−leucyl−L−phenylalanine in $10^{-6}$ M. A total of 0.9 ml of the PMN was added to each tube.

The resulting mixture was shaken for 10 minutes in a 37°C water bath. The reaction was stopped with 1.0 ml ice−cold 0.01 M phosphate−buffered 10% formaldehyde at pH 7.2. The contents of each tube were examined by phage microscopy and the cells from each tube were examined under 400 x magnification. The cells were counted and the percentage of total cells which polarized was determined. The stimulated cells had a very irregular shape compared to unstimulated cells which appeared to be circular.

Table I summarizes the data on the antibodies generated to date regarding the above procedures.

**TABLE I**

| Antibody | Isotype | Ascites C5a ELISA titer scale 2.0 (50 ng/well (C5a) | Affinity ($\ell$/mole)A | Affinity ($\ell$/mole)B | Ascites Dilution Giving 50% Inhibition of $^{125}$I-C5a Binding to Neutrophils | Purified Monoclonal Antibodies 10 µg/ml Percent Counts PPt. IP Score (Neg. control <3%) |
|---|---|---|---|---|---|---|
| 260-114G-10H-2G | IgG$_1$,K | 1:655,000 | $2.45\times10^8$ | $2.4\times10^9$ | 7000 | 33 |
| 260-91H-2G-10D | IgG$_1$,K | 1:328,000 | $2.18\times10^8$ | $4.4\times10^8$ | 7000 | 36 |
| 260-107B-3C-12C | IgG$_1$,K | 1:100,000 | $1.37\times10^8$ | $1.4\times10^7$ | 800 | 25 |
| 260-116G-5G-5H | IgG$_1$,K | 1:100,000 | | $4.4\times10^8$ | | 28 |
| 260-11H3-4A-11E | IgG$_1$,K | 1:100,000 | | | | 3 |
| 260-7C9-4G-10A | IgG$_1$,K | 1:100,000 | | $2.0\times10^8$ | | 29 |
| 261-174D-1B | IgG$_1$,K | 1:40,000 | $1.88\times10^8$ | $2.6\times10^8$ | 2000 | 29 |
| 261-9F11-6D | IgG$_1$,K | 1:200,000 | | $4.4\times10^8$ | | 34 |
| 269-10F7-4E-10A | IgG$_1$,K | 1:100,000 | | $3.3\times10^9$ | | 42 |
| 269-9H3-9C-6C | IgG$_1$,K | 1:2,000,000 | | $1.3\times10^9$ | | 29 |
| 269-3E5-7B-4G | IgG$_1$,K | 1:500,000 | | $2.4\times10^9$ | | 30 |
| 269-12F8-9A | IgG$_1$,K | 1:500,000 | | $4.4\times10^8$ | | 26 |
| Control (anti-TNF monoclonal antibody) | IgG$_1$,K | | | | | <3 |

Measurement of Binding in Molar Excess of C5

96 − well round − bottom vinyl assay plates were blocked for one hour with 1% PBS at 37°C. In the meantime, for activated sera each serum sample was incubated with 1:10 Saccharomyces cerivisceae cell wall extract capable of activating complement:serum, 50 mg extract/ml in $dH_2O$. The mixture was then stirred and incubated for 30 minutes at 37°C and then spun in Eppendorf centrifuge for three minutes. The pellet was discarded and the activated sera were removed to a new tube. A total of 50 $\mu$l of the sera was used neat for blocking each test sample. For tests using normal, nonactivated sera, 50 $\mu$l of neat sera (from the same source as that used for activation) was used to block.

For immunoprecipitation, 50 $\mu$l of the monoclonal antibody supernatant or purified monoclonal antibody was diluted in PBS + 1% BSA to give about 75% of maximum binding. Then 50$\mu$l of the neat sera (one each of the activated or non−activated sera) was added and incubation carried out at room temperature for one hour. Then 50 $\mu$l of iodinated C5a in PBS with 1% BSA was added to give about 100,000 counts per test. This solution was then incubated for one hour at room temperature. A total of 50 $\mu$l of iodinated C5a was saved to read as input counts.

Then a total of 50$\mu$l of rabbit anti−mouse immunobeads (1:5 stock beads in PBS) was added and the mixture was agitated for one hour at room temperature. The plates were spun two minutes at 2000 rpm to bring down the complex. The supernatant was aspirated with an 8−pronged manifold and then washed three times with a solution of PBS and 1% Tween 20 (200 $\mu$l), with careful aspiration each time. The wells were cut into tubes and the pellets read on a gamma counter. The percentage precipitated was determined by dividing counts bound by input counts x 100%.

The positive control employed was the antibody 260−114G with no serum, and the negative control was PBS/buffered saline and TNF ($IgG_1$,K) monoclonal antibody with and without serum.

For the antibody designated 260−11−46G, a graph of % input counts bound as a function of serum dilution (1/dilution) indicated that there was substantial binding of the antibody to C5a, which decreased as the antibody supernatant was diluted from 1:2 to 1:128. The binding of the TNF control was, by comparison, negligible as determined by % input counts bound.

In a test of animal sera blocking of the antibodies, there was no significant cross−reactivity with dog, monkey, pig or guinea−pig C5 or C5a as determined by immunoprecipitation inhibition of monoclonal antibodies plus $^{125}I$−C5a (human) using normal or activated animal sera. Test results of two antibodies, 260−114G and 260−7C9, are given in Table II.

TABLE II

**% of Input Counts Bound in Animal Sera**

| Serum Type and Antibody Supernatant | Dog | Monkey | Human | Pig | Guinea Pig | PBS S/N* 1:10 | PBS S/N* 1:30 |
|---|---|---|---|---|---|---|---|
| **260-11-4G** | | | | | | | |
| 1. Activated Serum (neat); Mab* S/N Dilution 1:10 | 18 | 21 / 3 | 19 | 25 | -- | | |
| 2. Nonactivated Serum (neat); Mab S/N Dilution 1:10 | 18 | 20 / 19 | 23 | 25 | -- | | |
| 3. Activated Serum (neat); Mab S/N Dilution 1:30 | 19 | 14 / 2 | 17 | 18 | -- | | |
| 4. Nonactivated Serum (neat); Mab S/N Dilution 1:30 | 14 | 20 / 10 | 19 | 18 | -- | | |
| 5. No Serum; Mab S/N Dilution 1:10 in PBS:BSA (1%) | -- | -- | -- | 19 | -- | | |
| 6. No Serum; Mab S/N Dilution 1:30 in PBS:BSA (1%) | -- | -- | -- | -- | 17 | | |
| **260-7C9** | | | | | | | |
| 1. Activated Serum (neat); Mab S/N Dilution 1:10 | 11 | 15 / 2 | 16 | 15 | -- | | |
| 2. Nonactivated Serum (neat); Mab S/N Dilution 1:10 | 13 | 17 / 14 | 16 | 7 | -- | | |
| 3. Activated Serum (neat); Mab S/N Dilution 1:30 | 7 | 5 / 1 | 6 | 7 | -- | | |
| 4. Nonactivated Serum (neat); Mab S/N Dilution 1:30 | 6 | 5 / 5 | 4 | 7 | -- | | |
| 5. No Serum; Mab S/N Dilution 1:10 in PBS:BSA (1%) | -- | -- | -- | 10 | -- | | |
| 6. No Serum: Mab S/N Dilution 1:30 in PBS:BSA (1%) | -- | -- | -- | -- | 5 | | |

*Mab and S/N refer to monoclonal antibody and supernatant, respectively.

The results show that the antibodies bind to human C5a in the presence of a molar excess of C5 from a wide variety of animal species, as indicated by the % of input counts bound for non – activated serum, which contains a molar excess of C5. The results also show that the antibodies inhibit C5a from humans but not from other animal species, as indicated by the differences between % of input counts bound for activated (C5a) and nonactivated serum (C5).

In vivo Studies

The anti – C5a antibody designated 260 – 91H – 2G – 10D and a control antibody which is not against C5a were injected intraveneously into two rabbits and allowed to equilibrate for 10 minutes. A blood sample was taken from each rabbit at Time 0 (before C5a was injected into each rabbit). Then 2.5 $\mu$g of C5a was injected intravenously into each rabbit and blood samples were taken one minute and five minutes after C5a injection (Times 1 and 5, respectively). Each blood sample was analyzed for total polymononucleocytes (PMN) and total white blood cells (WBC). The differential between total WBC and leukocyte (% PMN) was calculated. The results are given in Table III.

## TABLE III

| Antibody | WBC (per mm$^3$ blood)/%PMN | | |
|---|---|---|---|
| | 0 min. | 1 min. | 5 min. |
| Control monoclonal antibody which binds to ricin A chain | 9000/50 | 4000/1 | 5300/22 |
| Total PMN: | 4500 | 40 | 1166 |
| Anti-C5a monoclonal antibody | 8500/51 | 2300/15 | 4000/44 |
| Total PMN: | 4335 | 345 | 1760 |

The difference in total PMN at Times 1 and 5 between the control and anti–C5a antibodies indicates that the antibody herein blocks the effect of C5a in vivo.

Results in rabbits for seven other antibodies against C5a (68–2D3 being prepared by the same procedure as the others) are provided in Table IV, where 00 = prior to antibody iv injection, 0 = one hour after antibody injection and prior to C5a iv injection, 1 = one minute after C5a injection, and 5 = five minutes after C5a injection.

## TABLE IV

| Antibody | | WBC (per mm$^3$ blood)/%PMN | | | |
|---|---|---|---|---|---|
| | | 00 | 0 | 1 | 5 |
| 269-10F7 | | 5000/25 | 6000/38 | 4900/36 | 5700/34 |
| | Total PMN: | 12502280 | 1764 | 1938 | |
| 260-11H3 | | 4600/25 | 3700/34 | 1600/2 | 2900/16 |
| | Total PMN: | 11501258 | 32 | 464 | |
| 68-2D3 | | 3500*/22 | 2800*/43 | 1300*/2 | 2300*/20 |
| | Total PMN: | 7701204 | 26 | 450 | |
| 260-7C9 | | 5400/40 | 4600*/40 | 2700/32 | 5000/38 |
| | Total PMN: | 21601840 | 864 | 1900 | |
| 261-9F11 | | 6400/54 | 5200/46 | 4400/34 | 4500/32 |
| | Total PMN: | 34562392 | 1496 | 1440 | |
| 260-11G5 | | 6800/52 | 6600/50 | 5200/38 | 6400/40 |
| | Total PMN: | 35362300 | 1976 | 2560 | |
| 269-12F8 | | 4300/30 | 3200/38 | 1600/2 | 2300/18 |
| | Total PMN: | 12901216 | 32 | 414 | |

*Blood sample has a small dot.

EXAMPLE 2

Human antibodies against human des – arg74 – C5a are expected to be prepared by the following procedure.

Purification of Antigen

Human des – arg74 – C5a is purified from complement – activated serum to produce sufficient quantities for screening and in vitro stimulation of human B lymphocytes in preparation for generating human monoclonal antibodies.

Initially, modification of a C5a purification procedure described by Fernandez and Hugli, J. Immunol., 117:1688 (1976) will be employed. Part of the purified des – arg74 – C5a is used to immunize rabbits, to raise polyclonal antisera to be used in the immunoadsorbent purification scheme of Manderino et al., J. Immunol. Meth., 53:41 (1982). Briefly, human serum is incubated at 37°C for 60 minutes with boiled yeast cells (20 g/l) to activate complement. The yeast is removed by centrifugation and complement is heat – activated by incubation of the serum for 30 minutes at 56°C. The activated serum is applied to an immunoadsorbent column (Sepharose 6B – anti – des – arg74 – C5a) at a flow rate of 100 ml/hour at 4°C. The column is washed with PBS, and the adsorbed material is eluted with 200 mM glycine – HCl buffer, pH 2.8. After dialysis and concentration, gel filtration on Sephadex G – 75 is performed, and the fractions of molecular weight 10,000 – 15,000 daltons which possess chemotactic activity are pooled and concentrated.

Production of Antibodies

Peripheral blood B lymphocytes and/or human splenocytes are panned on purified des – arg74 – C5a, as described by Kozbar et al., Scan. J. Immunol., 10, 187 – 194 (1979) and Steinitz et al., J. Clin. Lab.

13

Immun., 2, 1 – 7 (1979), EBV transformed as described by Foung et al., J. Immun. Meth., 70, 83 – 90 (1984), and cultured in the presence of IL – 2 and other adjuvants. Fusions with human/mouse fusion partner F3B6 (ATCC #HB8785) are performed using standard techniques. Hybridoma supernatants are screened for binding to the purified des – arg74 – C5a. Positive hybrids are cloned by limiting dilution and hybridomas selected by standard techniques may be adapted to defined serum – free media such as HL – 1 for scale – up.

Of the murine hybrids, 260 – 11 – 4G – 10H – 2G and 260 – 9 – 1H – 2G – 10D were deposited at the American Type Culture Collection (ATCC), 12301 Parklawn Drive, Rockville, Maryland, USA. Deposit dates and accession numbers are given below. These hybrids have also been deposited with the Tissue Culture Collection (CTCC) of Cetus Corporation, Emeryville, CA, USA, the assignee of the present application, and assigned the indicated CTCC deposit numbers.

| Hybridoma | ATCC Deposit Date | ATCC Accession No. | CTCC Deposit No. |
|---|---|---|---|
| 260-114G-10H-2G | January 14, 1986 | HB8991 | 10,159 |
| 260-91H-2G-10D | January 14, 1986 | HB8992 | 10,160 |
| 269-3E5-7B-46-1B-2F | December 17, 1986 | HB9295 | 10,318 |
| 269-10F7-4G-10H-10A-46 | December 17, 1986 | HB9296 | 10,316 |
| 261-9F11 | December 30, 1986 | HB9302 | 10,319 |

The deposits above were made under the terms of the Budapest Treaty on the deposit of microorgan – isms for patent purposes pursuant to a contract between the ATCC and the assignee of this patent application, Cetus Corporation. The assignee of the present application has agreed that if the cell lines on deposit should die or be lost or destroyed when cultivated under suitable conditions, they will be promptly replaced on notification with a viable culture of the same cell line.

The foregoing written specification is considered to be sufficient to enable one skilled in the art to practice the invention. The present invention is not to be limited in scope by the cell lines deposited, since the deposited embodiment is intended as a single illustration of one aspect of the invention and any microorganisms which are functionally equivalent are within the scope of this invention. The deposit of materials herein does not constitute an admission that the written description herein contained is inadequate to enable the practice of any aspect of the invention, including the best mode thereof, nor are they to be construed as limiting the scope of the claims to the specific illustrations which they represent. Indeed, various modifications of the invention in addition to those shown and described herein will become apparent to those skilled in the art from the foregoing description and fall within the scope of the appended claims.

### Claims

1. A monoclonal antibody which binds with an affinity of at least $10^8$ liters/mole to human complement component C5a or des – arg74 – C5a in the presence of a molar excess of complement component C5 and blocks the binding of human C5a or human des – arg74 – C5a to human granulocytes.

2. An antibody as claimed in claim 1 which is of the isotype IgM or IgG.

3. An antibody as claimed in claim 1 or claim 2 and of murine origin or human origin.

4. An antibody as claimed in any one of claims 1 to 3 wherein the antibody is produced by a self – reproducing carrier cell containing genes coding for and specifying the production of the antibody.

5. An antibody of claim 4 wherein the cell has the characteristics of the hybridomas as exemplified by ATCC HB8991 and HB8992.

6. A composition for treating a condition associated with injurious introvascular complement activation comprising a therapeutically effective amount of at least one antibody as claimed in any one of claims 1 to 5 in an acceptable vehicle, or a therapeutically effective amount of at least two of said antibodies, in a parenterai vehicle each of said antibodies binding to different epitopes of human C5a or human

EP 0 245 993 B1

des – argf4 – C5a are employed.

**7.** An antibody as defined in any one of claims 1 to 5 or a composition as defined in claim 6 for use in a method of prophylactically or therapeutically treating a mammalian patient, e.g. a human patient, for a condition associated with injurious intravascular complement activation or the use of an antibody as defined in any one of claims 1 to 5 in preparing, producing or providing a medicament for a method as aforesaid.

**8.** An antibody or use as claimed in claim 7 wherein the condition is Gram – negative sepsis, adult respiratory distress syndrome, thermal injury, pulmonary inflammation or injury, severe trauma, blood transfusion, blood clots, systemic lupus erythermatosus, rheumatoid arthritis, pancreatitis, myocardial infarction, and exposure to medical devices.

**9.** A composition for treating Gram – negative sepsis comprising a therapeutically effective amount of a mixture of at least one antibody as claimed in any one of claims 1 to 5 and at least one antibody which is reactive with Gram – negative bacterial endotoxin, said antibody reactive with Gram – negative bacterial endotoxin being a monoclonal antibody which blocks the adverse biological effects of Gram – negative bacterial endotoxin, said mixture of antibodies being in a therapeutically acceptable vehicle.

**10.** An antibody as defined in any one of claims 1 to 5 or a composition as defined in claim 9 for use in a method of prophylactically or therepeutically treating a mammalian patient for Gram – negative sepsis by administering the antibody or composition to a patient, e.g. a human patient, or the use of an antibody as defined in any one of claims 1 to 5 or a composition as defined in claim 9 for use in preparing, producing or providing a medicament for a method as aforesaid.

**11.** An improved immunologic or immunodiagnostic method which comprises using at least one of the antibodies of any one of claims to 1 to 5 to detect human C5a or human des – arg74 – C5a in a fluid.

**12.** An immunologic or immunodiagnostic composition for detecting human C5a or human des – arg74 – C5a in a fluid, which composition comprises at least in part one or more antibodies of any of claims 1 to 5.

**13.** A hybridoma capable of secreting an antibody as defined in any one of claims 1 to 5.

**14.** A hybridoma of claim 13 and having the characteristics of a hybridoma exemplified by ATCC HB 8991 or HB 8992.

**15.** A method of producing an antibody as defined in claim 1 which comprises culturing a hybridoma as defined in claim 13 or claim 14.

**16.** A method of producing a medicament which comprises formulating for pharmaceutical or veterinarily use, optionally together with an acceptable vehicle, an antibody as defined in any one of claims 1 to 5.

**Patentansprüche**

**1.** Monoclonaler Antikörper, der in Gegenwart eines molaren Überschusses der Complement – Komponente C5 mit einer Affinität von wenigstens $10^8$ Litern/Mol die menschliche Complement – Komponente C5a oder des – Arg74 – C5a bindet und die Bindung von menschlichem C5a oder menschlichem des – Arg74 – C5a an menschliche Granulocyten blockiert.

**2.** Antikörper nach Anspruch 1, der den Isotyp IgM oder IgG aufweist.

**3.** Antikörper nach Anspruch 1 oder 2, der menschlichen oder Mäuse – Ursprungs ist.

**4.** Antikörper nach einem der Ansprüche 1 bis 3, wobei der Antikörper durch eine sich selbst fortpflan – zende Trägerzelle gebildet wird, die die Antikörperbildung codierende und spezifizierende Gene enthält.

**5.** Antikörper nach Anspruch 4, wobei die Zelle die Merkmale der Hybridome aufweist, für die ATCC HB8991 und HB8992 ein Beispiel sind.

**6.** Zusammensetzung zur Behandlung eines mit gesundheitsschädlicher, intravaskulärer Complement — Aktivierung verbundenon Zustandes, enthaltend eine therapeutisch wirksame Menge wenigstens eines Antikörpers nach einem der Ansprüche 1 bis 5 in einem verträglichen Vehikel oder eins therapeutisch wirksame Menge wenigstens zwei der genannten Antikörper in einem parenteralen Vehikel, wobei die Antikörper jeweils an unterschiedliche Epitope von menschlichem C5a oder menschlichem des — Arg74 — C5a binden.

**7.** Antikörper nach einem der Ansprüche 1 bis 5 oder Zusammensetzung nach Anspruch 6 zur Verwen — dung in einem Verfahren zur prophylaktischen oder therapeutischen Behandlung eines mit gesund — heitsschädlicher, intravaskulärer Complement — Aktivierung verbundenen Zustandes eines kranken Säugers, z.B. eines menschlichen Patienten oder Verwendung eines Antikörpers nach einem der Ansprüche 1 bis 5 zur Vorbereitung, Herstellung oder Bereitstellung eines Arzneimittels für ein vorgenanntes Verfahren.

**8.** Antikörper oder Verwendung nach Anspruch 7, wobei der Zustand eine gram — negative Sepsis, ein Atemnot — Syndrom bei Erwachsenen, eine thermale Verletzung, eine Lungenentzündung oder — verletzung, ein schweres Trauma, eine Bluttransfusion, ein Blutgerinnsel, ein systemischer Lupus erythematodes, eine rheumatische Gelenkentzündung, eine Bauchspeicheldrüsenentzündung, ein myocardialer Infarkt oder Exposition gegenüber medizinischen Vorrichtungen ist.

**9.** Zusammensetzung zur Behandlung gram — negativer Sepsis enthaltend eine therapeutisch wirksame Menge eines Gemisches von wenigstens einem Antikörper nach einem der Ansprüche 1 bis 5 und wenigstens einen Antikörper, der mit gram — negativem bakteriellem Endotoxin reagiert, wobei der mit gram — negativem, bakteriellem Endotoxin reagierende Antikörper ein monoclonaler Antikörper ist, der die nachteiligen, biologischen Wirkungen des gram — negativen, bakteriellen Endotoxins blockiert und wobei sich das Gemisch der Antikörper in einem therapeutisch verträglichen Vehikel befindet.

**10.** Antikörper nach einem der Ansprüchs 1 bis 5 oder Zusammensetzung nach Anspruch 9 zur Verwen — dung in einem Verfahren zur prophylaktischen oder therapeutischen Behandlung gram — negativer Sepsis eines kranken Säugers, wobei der Antikörper oder die Zusammensetzung dem Patienten, z.B. einem menschlichen Patienten, verabreicht wird oder Verwendung eines Antikörpers nach einem der Ansprüche 1 bis 5 oder einer Zusammensetzung nach Anspruch 9 zur Verwendung bei der Vorberei — tung, Herstellung oder Bereitstellung eines Arzneimittels für ein vorgenanntes Verfahren.

**11.** Verbessertes immunologisches oder immundiagnostisches Verfahren, wobei wenigstens einer der Antikörper nach einem der Ansprüche 1 bis 5 verwendet wird, um menschliches C5a oder menschli — ches des — Arg74 — C5a in einer Flüssigkeit nachzuweisen.

**12.** Immunologische oder immundiagnostische Zusammensetzung zum Nachweis von menschlichem C5a oder menschlichem des — Arg74 — C5a in einer Flüssigkeit, wobei die Zusammensetzung wenigstens teilweise einen oder mehrere Antikörper nach einem der Ansprüche 1 bis 5 enthält.

**13.** Hybridom, das zur Sekretion eines Antikörpers nach einem der Ansprüche 1 bis 5 in der Lage ist.

**14.** Hybridom nach Anspruch 13, das die Merkmale der Hybridome aufweist, für die ATCC HB8991 oder HB8992 ein Beispiel sind.

**15.** Verfahren zur Herstellung eines Antikörpers nach Anspruch 1, wobei ein Hybridom nach Anspruch 13 oder 14 kultiviert wird.

**16.** Verfahren zur Herstellung eines Arzneimittels, umfassend das Formulieren eines Antikörpers nach einem der Ansprüchs 1 bis 5 zur pharmazeutischen oder tiermedizinischen Verwendung, gegebenen — falls zusammen mit einem verträglichen Vehikel.

EP 0 245 993 B1

**Revendications**

1.  Anticorps monoclonal qui se lie, avec une affinité d'au moins $10^8$ litres/mole, au composant C5a du complément humain ou à la des – arg74 – C5a, en présence du composant C5 du complément en excès molaire, et qui bloque la fixation de la C5a humaine ou de la des – arg74 – C5a humaine aux granulocytes humains.

2.  Anticorps tel que revendiqué dans la revendication 1, d'isotype IgM ou IgG.

3.  Anticorps tel que revendiqué dans la revendication 1 ou la revendication 2, et d'origine humaine ou d'origine murine.

4.  Anticorps tel que revendiqué dans l'une quelconque des revendications 1 à 3, l'anticorps étant produit par une cellule hôte auto – reproductrice contenant des gènes qui codent pour l'anticorps et détermi – nent sa production.

5.  Anticorps de la revendication 4, la cellule présentant les caractéristiques des hybridomes dont des exemples sont ATCC HB8991 et HB8992.

6.  Composition pour traiter un état associé à l'activation intravasculaire nocive du complément, compre – nant une quantité efficace, du point de vue thérapeutique, d'au moins un anticorps tel que revendiqué dans l'une quelconque des revendications 1 à 5 dans un véhicule acceptable, ou une quantité efficace, du point de vue thérapeutique, d'au moins deux desdits anticorps, dans un véhicule parentéral, chacun desdits anticorps se liant à divers épitopes de la C5a humaine ou de la des – arg74 – C5a humaine.

7.  Anticorps tel que défini dans l'une quelconque des revendications 1 à 5, ou composition telle que définie dans la revendication 6, à utiliser dans une méthode de traitement prophylactique ou thérapeu – tique d'un patient mammifère, par exemple, un patient humain, présentant un état associé à l'activation intravasculaire nocive du complément, ou utilisation d'un anticorps tel que défini dans l'une quelconque des revendications 1 à 5 pour préparer, produire ou fournir un médicament destiné à une méthode telle que citée précédemment.

8.  Anticorps ou utilisation tels que revendiqués dans la revendication 7, l'état étant une septicémie gram – négative, un syndrome de détresse respiratoire de l'adulte, une lésion thermique, une lésion ou une inflammation pulmonaire, un traumatisme grave, une transfusion sanguine, des caillots sanguins, un lupus érythémateux systémique, une arthrite rhumatoïde, une pancréatite, un infarctus du myocarde et une exposition aux instruments médicaux.

9.  Composition pour traiter la septicémie gram – négative, comprenant une quantité efficace, du point de vue thérapeutique, d'un mélange composé d'au moins un anticorps tel que revendiqué dans l'une quelconque des revendications 1 à 5 et d'au moins un anticorps réactif avec l'endotoxine bactérienne gram – négative, ledit anticorps réactif avec l'endotoxine bactérienne gram – négative étant un anticorps monoclonal qui bloque les effets biologiquement négatifs de l'endotoxine bactérienne gram – négative, ledit mélange d'anticorps étant présent dans un véhicule acceptable, du point de vue thérapeutique.

10. Anticorps tel que défini dans l'une quelconque des revendications 1 à 5, ou composition telle que définie dans la revendication 9, à utiliser dans une méthode de traitement prophylactique ou thérapeu – tique d'un patient mammifère atteint de septicémie gram – négative, par administration de l'anticorps ou de la composition à un patient, par exemple, un patient humain, ou utilisation d'un anticorps tel que défini dans l'une quelconque des revendications 1 à 5 ou d'une composition telle que définie dans la revendication 9 pour préparer, produire ou fournir un médicament destiné à une méthode telle que citée précédemment.

11. Méthode immunologique ou méthode d'immunodiagnostic améliorée, comprenant qu'on utilise au moins un des anticorps de l'une quelconque des revendications 1 à 5 pour déceler la C5a humaine ou la des – arg74 – C5a humaine dans un liquide.

17

12. Composition immunologique ou composition d'immunodiagnostic pour déceler la C5a humaine ou la des − arg74 − C5a humaine dans un liquide, laquelle composition comprend, au moins en partie, un ou plusieurs des anticorps de l'une quelconque des revendications 1 à 5.

13. Hybridome capable de sécréter un anticorps tel que défini dans l'une quelconque des revendications 1 à 5.

14. Hybridome de la revendication 13 et possédant les caractéristiques d'un hybridome dont des exemples sont ATCC HB 8991 ou HB 8992.

15. Procédé pour produire un anticorps tel que défini dans la revendication 1, qui comprend qu'on cultive un hybridome tel que défini dans la revendication 13 ou la revendication 14.

16. Procédé pour fabriquer un médicament, qui comprend qu'on formule un anticorps tel que défini dans l'une quelconque des revendications 1 à 5, éventuellement associé à un véhicule acceptable, pour l'utilisation pharmaceutique ou vétérinaire.